(19) Europäisches Patentamt
European Patent Office
Office européen des brevets

(11) Publication number: 0 319 576 B1

# (12) EUROPEAN PATENT SPECIFICATION

(45) Date of publication of patent specification: 08.01.92 Bulletin 92/02

(51) Int. Cl.$^5$: C07C 405/00, A61K 31/557

(21) Application number: 88908640.1

(22) Date of filing: 10.02.88

(86) International application number: PCT/IT88/00010

(87) International publication number: WO 88/10252 29.12.88 Gazette 88/28

## (54) PROSTAGLANDIN DERIVATIVES, PROCESSES FOR PREPARING THEM AND PHARMACEUTICAL COMPOSITIONS CONTAINING SAID DERIVATIVES.

(30) Priority: 25.06.87 IT 2105587

(43) Date of publication of application: 14.06.89 Bulletin 89/24

(45) Publication of the grant of the patent: 08.01.92 Bulletin 92/02

(84) Designated Contracting States: AT BE CH DE FR GB IT LI LU NL SE

(56) References cited:
EP-A- 0 218 953
DE-A- 2 535 343

(73) Proprietor: IBI- Istituto Biochimico Italiano Giovanni Lorenzini S.p.A.
Via Lorenzini 2-4
Milan (IT)

(72) Inventor: VALCAVI, Umberto
Via Goldoni, 34
I-21100 Varese (IT)

(74) Representative: Klausner, Erich et al
c/o Ufficio Internazionale Brevetti Ing. C. Gregorj S.p.A. Via Dogana 1
I-20123 Milano (IT)

EP 0 319 576 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention relates to compounds having a prostaglandin -like structure and processes for the preparation thereof.

Furthermore, the instant invention relates also to pharmaceutical compositions having antiulcer activity.

The most modern treatments of peptic ulcers tend, on the one hand, to promote protective factors of the mucose whereas on the other they tend to moderate the acid or pepsine secretion, i.e. the aggressive agents.

Recently, some prostaglandin derivatives have been commercialized which possess both these characteristics: misoprostol (BE patent 827,127 of G.D.Searle & Co.,Chicago, Ill.) and, particularly in Italy, rosaprostol (IT patent 1,060,366 - issued on July 10,1982 owned by the Applicants).

The Applicants have also continued the study of new prostaglandine derivatives and have discovered that some functional changes to the structure of rosaprostol led to derivatives having a more potent citoprotective and anti-secretory activity than that possessed by the cited rosaprostol. These new discoveries are the subject matter of the IT patent application No. 22358 A/85.

Following along this route, it has now surprisingly been found that modifications in the cyclopentene ring and/or in the functional group at position 1 lead to derivatives possessing a high antiulcer activity.

These new compounds correspond to one of the following formulae:

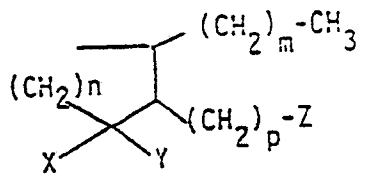

(IA)

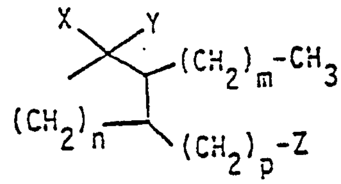

(IB)

wherein: n = 2, 3; m = 4, 5, 6, 7 ; p = 5, 6, 7; Y, in the case where X = H, $-NH_2$, represents $-NH-NH_2$, $-CH_2NH_2$, -NRR', wherin R and R' may be the same or different, each representing: H, a linear or branched alkyl, alkenyl, alkynyl, phenyl, substituted aryl; or,taken together with the substituent X, it may constitute the hydantoin ring (as in compounds (IIA) or (IIB), indicated purely for illustrative purposes).

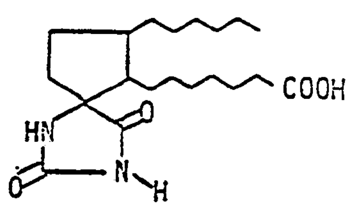

(IIA)

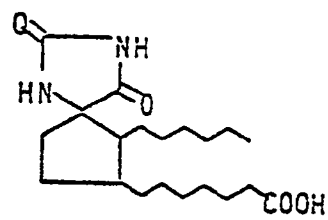

(IIB)

Z = -CN, $-CH_2NH_2$, $-CH_2OH$, -CHO, 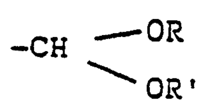 , $-COCH_3$,

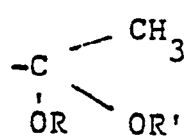 , COW,

wherin R and R' have the meanings given above and W may be -OR, -NRR'.

The compounds of formula (IA) wherein : n =2; m = 5; p = 6, X = H; Y = NRR' (R and R' having the meanings given above); and

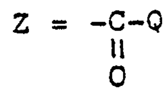

wherein Q = -OH, ($C_1$-$C_5$)-alkoxy, phenoxy, benzyloxy or NHR″ (wherein R″ is ($C_1$-$C_5$)-alkyl or H) are the subject matter of IT 1060366 and of the Italian patent application 22358 A/8(of the Applicants and therefore they are not claimed in the present application.)

The present invention includes also cationic pharmaceutically acceptable salts of the compounds of formula (IA) and (IB) when W = -OH and, in general, the pharmaceutically acceptable anionic salts of said compounds.

The expression "pharmaceutically acceptable cationic salts" refers to the alkali and alkaline earth metal salts such as, e.g. sodium, potassium, calcium, magnesium or salts of aluminum, ammonium, zinc and organic amines, such as e.g. triethanolamine, and also aminoacids such as e.g. lysine, arginine,phenylalaline and proline, inner salts and salts of basic resins.

The expression "pharmaceutically acceptable anionic salts" refers to salts obtained by the addition of an inorganic acid such as, e.g. hydrochloric, hydrobromic, nitric, phosphoric, sulphuric, or of an organic acid, such as e.g. benzene - sulfonic, benzoic, citric, laurylsulfonic, fumaric, oxalic, maleic, methanesulfonic, tartaric, ascorbic, p-toluenesulfonic, salicylic and succinic acid. With polybasic acids, the salt may include more than one mole of base per mole of acid. However, the salts obtained from one mole of acid per mole of the inventive compound are preferred.

The antiulcer activity of the compounds of the instant invention is evaluated by the ethanol induced ulcers, by the hydrochloric acid induced ulcers and by the influence of the gastric secretion in the rat.

The compounds of the present invention can be prepared according to the reactions known in the art and the reaction schemes of which are outlined hereinafter only for illustrative purposes.

1) The compounds of formula (IA) and (IB) wherein:

- n, m, p have the meanings given above,
- X = H, $NH_2$; Y = $NH$-$NH_2$, $CH_2NH_2$, NRR′, wherein R and R′ can be the same or different, each representing H, a linear or branched alkyl, alkenyl, alkynyl, substituted aryl,and
- Z has the meanings given above,

can be prepared starting from the ketones of formula (IIIA) and (IIIB) respectively, according to per se known reactions such as, e.g. reductive aminations (with $H_2$ and metal catalysts rather than with hydrides of different metals) or the introduction of $C_1$-units by means of the Wittig reaction using triphenyl-phosphonium methoxymethylide ($Ø_3P$=$CH$-$OCH_3$) , hydrolysis of vinyl ether, reduction to alcohol, preparation of the corresponding mesylate, reaction with $NaN_3$ and reduction to amine, or else by reducing the ketone to alcohol, preparing the mesylate, substituting with $NaN_3$ and reducing same. The reductive amination is shown in the following reaction schemes (schemes 1A and 1B).

SCHEME 1A

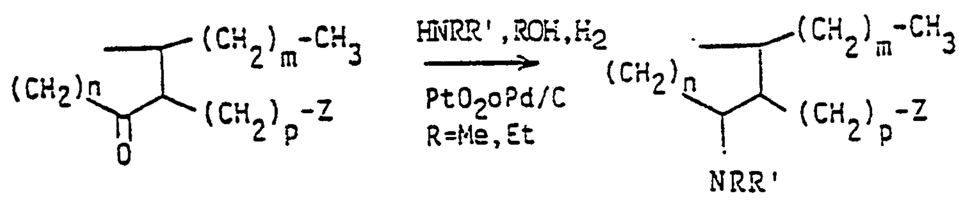


(IIIA) (IVA)

SCHEME 1B

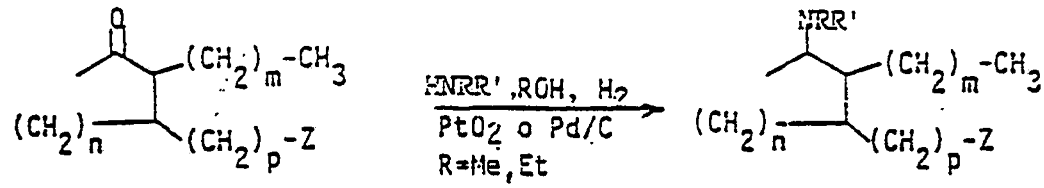


(IIIB) (IVB)

2) The compounds of formula(IA) and (IB) wherein X and Y, taken together, form ahydantoin ring, while n, m, p and Z have the meanings given above, are prepared from ketones (IIIA) and (IIIB) respectively by reacting with cyanide salts under controlled pH (reactions schemes 2A and 2B):

SCHEME 2A

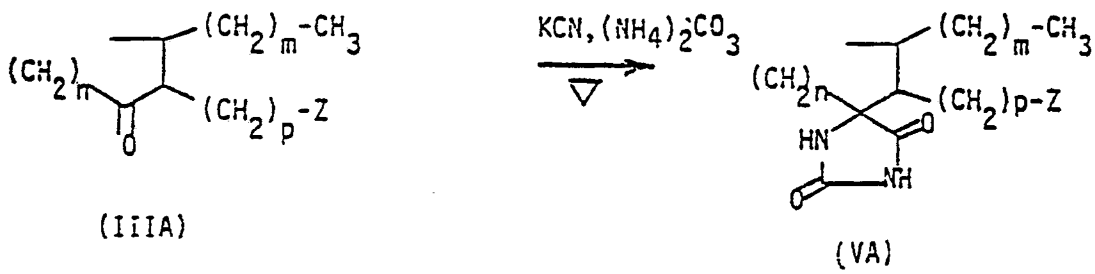


(IIIA) (VA)

SCHEME 2B

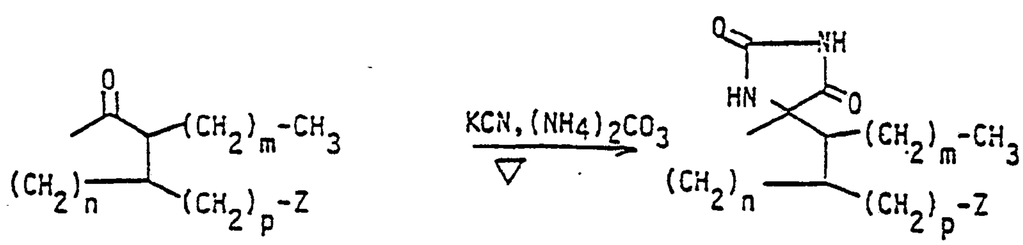


(IIIB) (VB)

The efficacy of the compounds of the instant invention as antiulcer agents has been determined by means of the following tests:

i) Ethanol induced ulcers in the rat

The tests were carried out according to what has been described in literature (A. Robert et al. Gastroenterology 77, 433 (1979) (Table 1)

ii) Hydrochloric acid induced ulcers in the rat

The tests were carried out according to what has been described in literature (A.Robert et al. Gastroenetrology 77, 433 (1979) ) (Table 2).

iii) Influence on the gastric secretion in the rat

The tests were carried out according to what has been described in literature (Shay et al. Gastroenterology

5, 43, (1945) (Table 3).

iv) Cytoprotective activity in the rat

The tests were carried out according to what has been described in literature:
1 (A.Robert et al. Gastroenterology, 77, 433 (1979)) (Table 4)
2 (Szabo et al. Sciene 214 (200) (1981)) (Table 4)

## TABLE 1

ETHANOL INDUCED ULCERS IN THE RAT

| | Doses | | |
|---|---|---|---|
| COMPOUND | 25 mg/Kg | 12,5 mg/Kg | 6,5 mg/Kg |
| IBI-P-01023 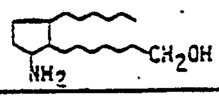 | 50 | 25 | 5 |
| IBI-P-01028 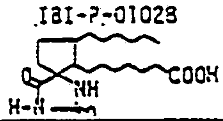 | 69 | 39 | 27 |
| IBI-P-01037 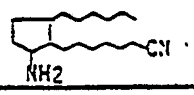 | 92 | 87 | 64 |
| IBI-P-01053 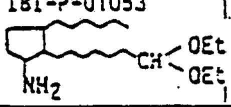 | 91 | 90 | 72 |
| IBI-P-01056 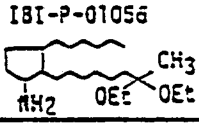 | 90 | 84 | 66 |
| IBI-P-01058 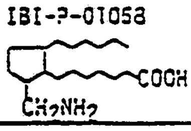 | 91 | 87 | 77 |
| IBI-P-01062 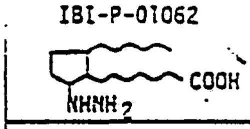 | 87 | 50 | 48 |
| IBI-P-01068 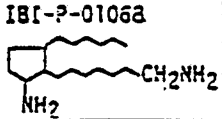 | 97 | 87 | 60 |
| IBI-P-01070 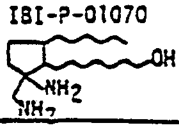 | 87 | 56 | 33 |
| IBI-P-05006 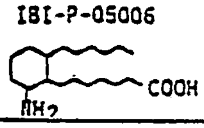 | 98 | 91 | 78 |
| IBI-P-05008 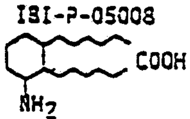 | -- | -- | 24 |

continuation T A B L E 1

| Compound | Doses | | |
|---|---|---|---|
| | 25 mg/Kg | 12,5 mg/Kg | 6,3 mg/Kg |
| IBI-P-05009 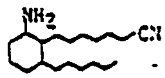 | -- | 55 | 52 |
| IBI-P-05010 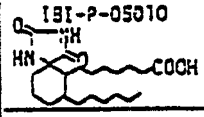 | 0 | -- | -- |
| IBI-P-05011 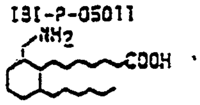 | 92 | 98 | 81 |
| IBI-P-05012 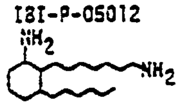 | 88 | 78 | 76 |
| IBI-P-10002 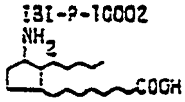 | 75 | 75 | 41 |
| IBI-P-10004 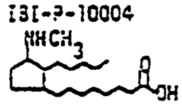 | 85 | 70 | 40 |
| IBI-P-10007 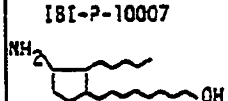 | 73 | 29 | 5 |
| IBI-P-12004 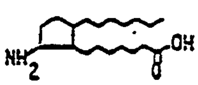 | 86 | 85 | 80 |
| IBI-P-12007 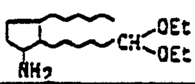 | 73 | 29 | 5 |
| IBI-P-12008 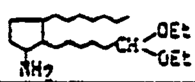 | 94 | 78 | 21 |
| IBI-P-12009 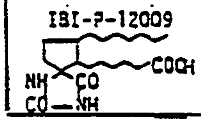 | 59 | 58 | 28 |

## TABLE 2

HYDROCHLORIC ACID INDUCED ULCERS IN THE RAT

| Compound | Doses 25 mg/Kg | Doses 12,5 mg/Kg | Doses 6,25 mg/Kg |
|---|---|---|---|
| IBI-P-01023 | 50 | 30 | 10 |
| IBI-P-01028 | 55 | 56 | 40 |
| IBI-P-01037 | 96 | 84 | 47 |
| IBI-P-01053 | 80 | 65 | 31 |
| IBI-P-01056 | 82 | 74 | 32 |
| IBI-P-01058 | 73 | 58 | 43 |
| IBI-P-01062 | 43 | 40 | 25 |
| IBI-P-01068 | 87 | 80 | 23 |
| IBI-P-01070 | 53 | 39 | 20 |
| IBI-P-05006 | 81 | 70 | 59 |
| IBI-P-05008 | 42 | 26 | 0 |
| IBI-P-05009 | 78 | 68 | 32 |
| IBI-P-05010 | 13 | 0 | 0 |
| IBI-P-05011 | 57 | 47 | 1 |
| IBI-P-05012 | 90 | 86 | 39 |
| IBI-P-10002 | 70 | 47 | 41 |
| IBI-P-10004 | 85 | 63 | 14 |
| IBI-P-10007 | 89 | 92 | 84 |
| IBI-P-12004 | 80 | 49 | 46 |
| IBI-P-12007 | 88 | 58 | 33 |
| IBI-P-12008 | 60 | 48 | 35 |
| IBI-P-12009 | 58 | 39 | 12 |

TABLE 3

Gastric Secretion after two hours

| Compound | Doses 100 mg/Kg | 50 mg/Kg | 25 mg/Kg |
|---|---|---|---|
| IBI-P-01023 | 48 | 20 | 5 |
| IBI-P-01028 | 82 | 69 | 45 |
| IBI-P-01037 | 83 | 83 | 56 |
| IBI-P-01053 | 83 | 73 | 74 |
| IBI-P-01056 | 69 | 60 | 51 |
| IBI-P-01058 | 88 | 70 | 40 |
| IBI-P-01062 | 71 | 28 | 0 |
| IBI-P-01063 | 94 | 93 | 85 |
| IBI-P-01070 | 90 | 83 | 65 |
| IBI-P-05006 | 56 | 50 | 6 |
| IBI-P-05008 | 72 | 46 | 0 |
| IBI-P-05009 | -- | 61 | 42 |
| IBI-P-05010 | 55 | 19 | 0 |
| IBI-P-05011 | 60 | -- | -- |
| IBI-P-05012 | 91 | 79 | 73 |
| IBI-P-10002 | 73 | 24 | 27 |
| IBI-P-10004 | 74 | 42 | 35 |
| IBI-P-10007 | 71 | 56 | 49 |
| IBI-P-12004 | 84 | 76 | 62 |
| IBI-P-12007 | 84 | 78 | 74 |
| IBI-P-12008 | 57 | 77 | 54 |
| IBI-P-12009 | 68 | 48 | 22 |

Cytoprotective activity $DE_{50}$ mg/kg TABLE 4

| Compound | oral $LD_{50}$ rat | Ethanol (1) +NEM | NaCl (2) 25% | NaOH (2) 0.2 N | Acetyl salicylic acid + HCl |
|---|---|---|---|---|---|
| IBI-P-01028 | 2000 mg/Kg | 30 | 15 | 18 | 78 |
| IBI-P-01053 | 3000 mg/Kg | 7 | 17 | 6 | 252 |
| IBI-P-05006 | 3000 mg/Kg | 20 | 25 | 9 | > 200 |
| IBI-P-10007 | 3000 mg/Kg | 8 | 4 | 4 | 165 |

The cytoprotective and antisecretative activity shown by the inventive compounds in the referred tests reflects the usefulness of the drugs subject of the present invention in the treatment of peptic ulcers in mammals, including man.

The compounds of the present invention are preferably administered as pharmaceutical compositions, in admixture with one or more pharmacologically acceptable diluents and/or excipients.

Within the scope of the present description the term "pharmacologically acceptable diluents and/or excipients" refers to substances such as,e.g. amides and derivatives thereof (maize and rice starch,carboxymethyl starch),cellulose and its derivatives(such as AVICEL®, registered trademark of FMC Corporation of Philadelphia,U.S.A., and methylcellulose, ethylcellulose, hydropropyl-methylcellulose); silicates ans silicone oxides (talc, hydrated calcium silicate, Mg and Al silicates,AEROSIL®, registered trademark of Wacker-Chemie GmbH, Munich,FRG,SYLOID ®, registered trademark of W.R.Grace & Co., New York, U.S.A.); solid ionic surfactants (sodium laurylsulphate) and non-ionic surfactants (such as esters of saccarose with fatty acids); acrylic derivatives and the polymers thereof; alkali and alkaline earth metal sulphates, carbonates and phosphates; polyvinyl pyrrolidone and derivatives thereof. They are preferably administered orally in the form of tablets, granules, syrups, etc. or perenterally (i.v. or i.m). Although the dosage can vary according to the symptoms, sex, body weight and conditions of the patient and also according to the frequency of the route of administration, the inventive compounds can be orally administered to an adult at a daily dosage of from 0.1 to 3000 mg,preferably at from 1 to 2000 mg in a single dose or in subdivided doses over 24 hours.

Example 1

2-(7-hydroxyheptyl)-3-(hexyl)-cyclopentylamine (formula (IA)),wherein n= 2, m = 5, p = 6, X = H, Y = $NH_2$, Z =$CH_2OH$; (compound IBI-P-01023).

A suspension of $LiAlH_4$ (2.3 g) in ethyl ether (400 ml) is added dropwise, under argon, with a solution of 2-(carobxylhexyl)-3-hexyl-cyclopentylamine (15 g) in ethyl ether (100 ml, keeping the temperature at +25°C. The reaction mixture is stirred for 3 hours. It is then cooled to 0°C and added dropwise with ethyl acetate (40 ml) and thereafter water (100 ml) is cautiously added whereupon the phases are separated. The organic phase is washed to neutrality and anhydrated,evaporated to dryness under reduced pressure,yielding the title compound (12.3 g).

Analysis:

I.R. (vmax) : 3340 $cm^{-1}$
$^1$H-NMR : 3.8 ÷ 3.3 (m, 3H); 2.1 (s, 3H); 1.8 ÷ 0. (broad, 31H )
Equivalent weight = 309.1 ( =96%)

| Elemental analysis: | | calculated | found |
|---|---|---|---|
| | C | 76.32 | 76.1 |
| | H | 13.07 | 12.96 |
| | N | 4.94 | 5.01 |

Example 2

5-spiro-[2-(6-carboxy-ethyl)-3-hexyl-cyclopentyl]-imidazoline-2,4-dione (formula (IA)), wherein n= 2, m = 5, p = 6; X and Y represent the hydantoin nucleus; Z = COOH; (IBI-P-01028).

A solution of KCN (18 g) and $(NH_4)_2CO_3$ (84 g) in water (450 ml) is added with a solution of the sodium salt of 9-oxo-bis-nor-prostanoic acid (90 g) in water (450 ml). The reaction mixture is heated for 5 hours at 80°C under stirring. It is then cooled to 0 ÷ 5°C and acidified with 6N HCl at pH = 2. The acid solution is extracted with ethyl ether (300 ml x 3), washed with water to neutrality and anhydrated. The solution is evaporated to dryness whereupon a semi-solid is obtained which is washed repeatedly with hexane and crystallized from 8 : 2 ethyl ether/hexane. 55 g of white crystalline compound is obtained.

Analysis:

I.R.:3250,3200,3140,3020, 1700, 1750, 1720,1675, 1460 $cm^{-1}$

$^1$H-NMR: 9.4 (s, 1H); 7.3 (s, 1H); 2.35 (m, 4H); 1.4(broad,24 H); 0.95 (t. 3H)

Example 3

2-(7-hydroxy-heptyl)-3-hexyl-cyclopentanol

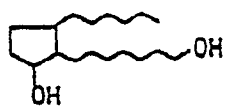

A solution of 9-oxo-bis-nor-prostanoic acid (308 g) in THF (3 l) is added with triethylamine (168.5 ml). The reaction mixture is brought,under stirring,to -30°C and added dropwise with ethyl chloroformate (109 ml) in 15 minutes whereby the temperature is allowed to rise to 0 ÷+5°C,then keeping said temperature for 30 minutes. The salts are filtrated by means of a Buchner filter and the filtrate is added dropwise into a solution of $NaBH_4$ (198 g) in water (2 l), while the temperature is kept at +10 ÷ 15°C,in about 30 minutes. At the end, the reaction mixture is stirred for 30 minutes at +15°C and the salts are filtered once more, the phases are separated and the liquor is extracted with ethyl ether (500 ml). Subsequently, the combined organic phases are washed with a saturated NCl solution and evaporated to dryness under reduced pressure. 238 g of a clear oil is obtained.
I.R. (ν max): 3330 $cm^{-1}$

Example 4

2-(7-oxo-heptyl)-3-hexyl-cyclopentanol

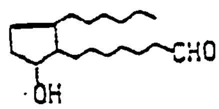

A solution of the compound of example 3 (100 g) in methylene chloride (360 ml) is added with KBr (1.15 g), 2,2,6,6-tetramethyl-piperidine-1-oxide (TEMPO,0.275 g). The reaction mixture is then cooled to -5°C and added dropwise with a 0.5M sodium hypochloride solution which has been previously saturated with $NaHCO_3$ (861 ml), keeping the temperature at +5 ÷+10°C. The reaction mixture is then left at 15°C for 1 hour and the phases are separated. The organic phase is washed with water, anhydrated and evaporated under reduced pressure. 102.9 g of a fluid oil is obtained.
I.R. (ν max ): 2270, 1720 $cm^{-1}$

Example 5

3-hexyl-2-(6-cyano-ethyl)cyclopentanol formate

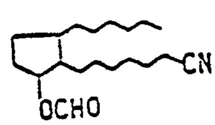

A solution of the compound according to Example 4 (102 g) in HCOOH (500 ml) is added at room temperature with hydroxylamine hydrochloride (32 g). The reaction mixture is then heated to 50°C and stirred for 30 minutes. It is evaporated under reduced pressure to a small volume and poured into water (800 ml), added with ethyl ether (800 ml) and the pH is adjusted to neutrality with 10% NaOH. The ethereal phase is separated and washed with water to neutrality. It is then anhydrated and evaporated, yielding a dense dark oil (100 g). Said oil is taken up with formic acid (600 ml) and heated at 40°C for 40 minutes. The reaction mixture is then evaporated to dryness,under reduced pressure, it is taken up with ethyl ether(500 ml) and washed with a water/ saturated sodium chloride solution up to neutrality. The reaction mixture is anhydrated and the solvent is evaporated. A yield of 105 g (brown oil) is obtained.
I.R. (ν max ): 2220, 1740 $cm^{-1}$

Example 6

3-hexyl-2-(6-cyano-hexyl)-cyclopentanol

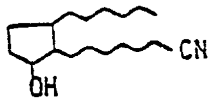

The compound of Example 5 (105 g) dissolved in methanol (1500 ml) is added with a $K_2CO_3$ (44 g) solution in water (250 ml) and stirred for 40 minutes. The reaction mixture is concentrated to a small volume, under reduced pressure, added with water and extracted with ethyl ether (500 ml). The extract is washed with water up to neutrality,it is then anhydrated and the solvent is evaporated. 96 g of a dense oil is obtained which is then chromatographed on silica gel, eluting with a 7 : 3 n-hexane/ethyl acetate mixture, thus yielding 81 g of the title compound.

Analysis:

I.R. (ν max): 3400, 2220 $cm^{-1}$
$^1$H-NMR : 4.2 (m, 1H); 2.3 (t, 2H); 2 (s,1H); 0.8 (t,3H)

Example 7

3-hexyl-2-(6-cyano-hexyl)-cyclopentyl-azide

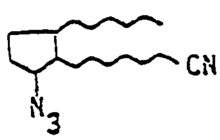

A solution of the compound according to Example 6 (60 g) in methylene chloride (600 ml) is added with triethylamine (45.5 ml). The reaction mixture is then cooled to 0°C and added dropwise with a mesylchloride solution (21 ml), in methylene chloride (100 ml) in 20 minutes. The reaction mixture is then kept 30 minutes at a temperature of 0÷+5°C and poured into water (800 ml). The phases are separated and the organic phase is washed with water to neutrality. It is anhydrated and evaporated yielding 78 g of a clear oil (I.R. = 2220 $cm^{-1}$). The oily residue is then taken up with water (160 ml), added with $NaN_3$ (35 g), hexadeciltributyl-fosfonium bromide (16 g) and stirred at 75°C for 4 hours. At the end, it is cooled and extracted with ethyl ether (500 ml). The organic phase is washed with water and anhydrated. The solvent is evaporated at reduced pressure, yielding 71 g of a dense oil. After silica gel chromatography, using a 9 : 1 hexane/ethyl acetate eluent, 40 g of the title compound is obtained.
I.R. (ν max) : 2100, 2200 $cm^{-1}$

Example 8

3-hexyl-2-(6-cyano-hexyl)-cyclopentylamine. Derivative of formula (IA) wherein : n = 2; m = 5; p = 6; X = H; Y $=NH_2$;
Z = CN (compound IBI-P-01037).

The compound according to Example 7 (20 g) dissolved in methanol (500 ml) is added with 5% palladium on carbon(2g). The reaction mixture is then subjected to hydrogen atmosphere and stirred for 4 hours at room temperature. At the end, the catalyst is filtered off and the solvent is evaporated under reduced Pressure. 18 g of the title compound is obtained.

Analysis

I.R. (ν max) : 3300, 2200 $cm^{-1}$
Titre as base = 95%

Elemental analysis:

| | calculated | found |
|---|---|---|
| C | 77.63 | 77.17 |
| H | 12.31 | 12.24 |
| N | 10.06 | 9.84 |

Example 9

3-hexyl-2-(6-diethoxymethyl-hexyl)-cyclopentanol

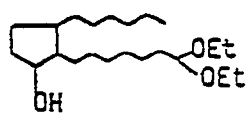

The compound according to Example 4(80 g), dissolved in ethanol (800 ml),is added with p-toluene sulfonic acid (2.5 g) and stirred for 1 hour at room temperature. The reaction mixture is poured into water (1 l) and 5% bicarbonate (100 ml) and extracted with ethyl ether (500 ml). The organic phase is separated and washed with water and anhydrated. Upon evaporation, 92 g of a fluid oil is obtained.
I.R. (ν max): 3300 $cm^{-1}$

Example 10

3-hexyl-2-(6-diethoxymethyl-hexyl)-cyclopentyl-azide

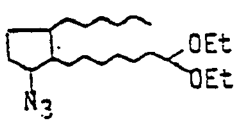

The compound according to Example 9 (29 g) is handled as described in Example 7 using methylene chloride (300 ml), triethylamine (25 ml), mesylchloride (14 ml), water (280 ml), $NaN_3$ (14.2 g), hexadecyl-tributyl-fosfonium bromide (4.4 g), obtaining thereby 27 g of the title compound.
I-R. (ν max) : 2100 $cm^{-1}$

Example 11

3-hexyl-2-(6-diethoxymethyl-hexyl)-cyclopentyl amine, the compound of formula (IA) wherein : n = 2; m = 5; p = 6; X = H; Y = $NH_2$; Z = $CH(OEt)_2$ (IBI-P-01053).

The compound of Example 10 (27 g) is handled as described in Example 8 using ethanol instead of methanol (500 ml) and 5% Pd/C (1.5 g).Then 22 g of a fluid oil is obtained which is subjected to silica gel chromatography,eluting with 8:2 $CH_2Cl_2$/ethanol,thus obtaining 14 g of the title compound.

Analysis

I.R. (ν max) : 3300, 2200 $cm^{-1}$
$^1$H-NMR: 4.2 (t,1H);3.5 (m, 5H); 2.2 ÷ 0.8 (m, 39 H)
Titre as base = 99%

| Elemental analysis: | | calculated | found |
|---|---|---|---|
| | C | 74.36 | 74.21 |
| | H | 12.67 | 12.33 |
| | N | 7.88 | 8.01 |

Example 12

3-hexyl-2-(7-hydroxy-octyl)-cyclopentyl-azide

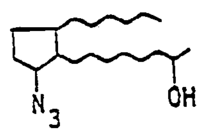

The compound of Example 10 (23 g) is added with acetone (350 ml) and 2N HCl (50 ml). The reaction mixture is stirred at room temperature for 1 hour. Thereafter, the reaction mixture is added with ethyl ether (300 ml) and water (500 ml). The phases are separated and the organic phase is washed with water to neutrality. The reaction mixture is anhydrated and evaporated under reduced pressure to give 18 g of an oily residue that is taken up with ethyl ether (180 ml) and treated with a solution of 0.5N $CH_3MgI$, in ether (120 ml) at 0°C. At the end of the addition, the reaction mixture is decomposed with slightly acidified water,ant the organic phase is washed with water. The reaction mixture is anhydrated and evaporated under reduced pressure. 14 g of a clear oil is obtained.
I.R. (ν max) : 3360, 2100 $cm^{-1}$

Example 13

3-hexyl-2-(7-keto-octyl)-cyclopentyl-azide

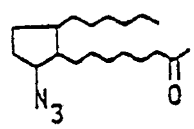

To the compound of Example 12 (10.2 g), dissolved in acetone (200 ml) is added dropwise an aqueous solution of the Jones reactive (12 ml) at 0°C,whereafter the reaction mixture is left 15 minutes at 0 ÷ +5°C and it is then poured into a 1 : 1 ethanol/water solution (200 ml). The phases are separated and the ethereal phase is washed with water up to neutrality.The solvent is anhydrated and evaporated. 9.4 g of a brown oil is obtained.
I.R. (ν max) : 2100, 1720 $cm^{-1}$

Example 14

3-hexyl-2-(7,7'-diethyoxy-octyl)-cyclopentyl-azide

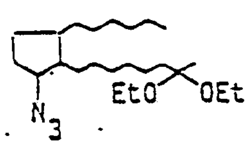

The compound of Example 13, dissolved in n-hexane (980 ml), is added with K 10 montmorillonite/triethylorthoformate (100 g),previously prepared by suspending the montmorillonite (60 g) in triethylorthoformate (70 ml) and filtering over Buchner the thus obtained complex which is used in the wet form. The reaction mixture is stirred for 2 hours at room temperature,and the solid is filtered off. The filtered organic phase is washed with

bicarbonate and anhydrated. It is evaporated to dryness yielding 10 g of a brown oil.
I.R. (ν max): 2100 $cm^{-1}$.

Example 15

3-hexyl-2-(7,7'-diethyoxy-octyl)-cyclopentylamine, derivative of formula (IA) wherein n= 2; m = 5; p = 6; X = H; Y = $NH_2$; Z = $-C(OC_2H_5)_2-CH_3$ (IBI-P-01056)

The compound of Example 14 (10 g) is handled as described in Example 8, using ethanol (150 ml), 5% Pd/C (2g). At the end of the operation, 8 g of the title compound is obtained.

Analysis:

I.R. (ν max) : 3340 $cm^{-1}$
$^1$H-NMR : 3.4 (m, 5H); 2 ÷ 0.8 (m, 42H)
Titer as base = 96%

| Elemental analysis: | | calculated | found |
|---|---|---|---|
| | C | 74.7 | 74.42 |
| | H | 12.81 | 12.66 |
| | N | 3.79 | 3.91 |

Example 16

3-hexyl-2-(6-carbomethoxy-hexyl)-hydroxymethyl-cyclopentane

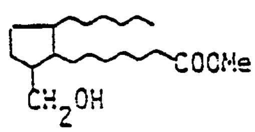

Potassium ter-butylate (30 g) is added in one go to anhydrous DMSO (3 l) and stirred until it is completely dissolved, whereupon it is added with methoxymethyl-triphenyl-phosphonium chloride (91.6 g). The solution is kept 15 minutes at room temperature. The methyl ester of 9-oxo-nor-prostanoic acid is then rapidly added and the reaction mixture is stirred at room temperature. It is then poured into water (6 l) and 1N HCl (0.6 l), extracted with ethyl ether (2 x 4 l) and the ethereal phase is washed with water to neutrality. The reaction mixture is anhydrated and evaporated at reduced pressure. The thus obtained residue (111 g) is purified by means of silica gel chromatography eluting with 9 : 1 n-hexane/ethyl acetate, thus obtaining 31 g of a brown dense oil. Said substance is taken up with acetone (1 l) and 3N HCl (200 ml) and stirred for 1 hour at room temperature. The acetone is evaporated under reduced pressure and the reaction mixture is thereafter extracted with methylene chloride, the organic phase is washed with water to neutrality and evaporated at reduced pressure, to give 49 g of a brown oily residue. The thus obtained residue is dissolved in methanol (500 ml) and treated with $NaBH_4$ (7g) for 1 hour at 0°C. The reaction mixture is evaporated at reduced pressure, up to a small volume, it is added with slightly acid water and extracted with ethyl ether (400 ml). The etheral phase is washed with water and anhydrated. It is evaporated,at reduced pressure,yielding 32 g of the title compound.
I.R. (ν max): 3200, 1740 $cm^{-1}$

Example 17

3-hexyl-2-(6-carbomethoxy-hexyl)-azidomethyl-cyclopentane

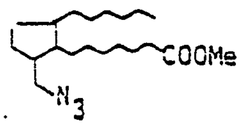

The compound of Example 16 (32 g) is handled as described in Example 7 using $CH_2Cl_2$ (300ml),$N(C_2H_5)_3$ (21.5 ml), mesyl chloride (15ml), water (450 ml) $NaN_3$ (16.6 g), hexadecil-tributylphosphonium bromide (5.1 g).

At the end of the reaction, 30 g of the chromatographed compound is obtained.

I.R. (ν max) : 2100; 1740 $cm^{-1}$

Example 18

3-hexyl-2-(6-carbomethoxy-hexyl)-aminomethyl-cyclopentane

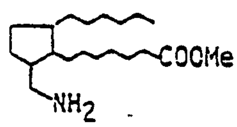

The compound of Example 17 (30 g) is handled as described in Example 8, using methanol (30 g) 5M Pd/C (10 g). 15.1 g of the compound, chromatographed on silica gel, is obtained and eluted with 8 : 2 n-hexane/ether.

I.R. (ν max) : 3300, 1740 $cm^{-1}$

Example 19

3-hexyl-2-(6-carboxy-hexyl)-aminomethyl-cyclopentane, compound of formula (IA) wherein n = 2; m = 5; p = 6; X = H; Y = $CH_2NH_2$; Z=COOH (IBI-P-01058).

The compound of Example 18 (6.6 g), dissolved in methanol (80 ml),is handled with a solution of $K_2CO_3$ (5.6 g) in water (30 ml) and refluxed for 3 hours. The methanol is evaporated and the solution diluted with water (50 ml) and then the pH adjusted to 5.8 with 6N HCl. The solid white precipitate is filtered off and dessicated under reduced pressure.

5.2 g of the white crystalline title compound is obtained.

I.R. (ν max) : 2700, 1625, 1530.

Titre as base= 93%

| Elemental analysis : | | calculated | found |
|---|---|---|---|
| | C | 73.26 | 73.19 |
| | H | 11.97 | 11.95 |
| | N | 4.50 | 4.62 |

Example 20

N-[3-hexyl-2-(6-carboxy-hexyl)-cyclopentyl]-N'-(terbutoxy-carbonyl))-hydrazine

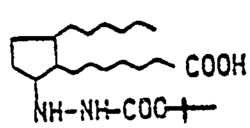

A solution of 9-oxo-19,20-bis-nor-prostanoic acid (29.6 g) in methanol (100 ml) is added with Boc-hydrazine (16 g) dissolved in methanol (100 ml) and stirred for 1 hour at 30°C. Water (400 ml) is added and the phases extracted with ether (400 ml). The etheral phase is washed with water (200 ml) and anhydrated. After evaporation, 43.8 g of a solid is obtained with melting point at 107°C. This substance is redissolved in methanol (400

ml) and treated with $PtO_2$ (2 g) in a small hydrogenator. The reaction mixture is kept 6 hours under a hydrogen pressure of 4÷5 atm. at 60°C. The catalyst is filtered off and the solvent evaporated. The thus obtained crude compound (44.6 g) is chromatographed on silica gel, eluting with 1 : 1 ether/n-hexane. The title compound (35 g) is obtained as a white waxy solid.

Analysis:

I.R. (ν max): 3300, 1710 $cm^{-1}$
$^1$H-NMR : 1.4 (S; 9H)

| Elemental analysis: | | calculated | found |
|---|---|---|---|
| | C | 66.95 | 66.81 |
| | H | 10.75 | 10.86 |
| | N | 6.79 | 6.55 |

Example 21

[3-hexyl-2-(6-carboxy-hexyl)]-hydrazine hydrochloride, compound of formula (IA) wherein: n = 2; m = 5; p = 6; X = H; Y = NH-$NH_2$; Z = COOH (IBI-P-01062).

Into a solution of the compound of Example 20(27.5 g),in ethyl acetate (400 ml), cooled to 0°C, is bubbled gaseous HCl. The reaction mixture is stirred for 3 hours at room temperature , evaporated to dryness and taken up with deionized water (500 ml) and ethyl ether (300 ml). The phases are separated and the aqueous phase is evaporated to dryness. The thus obtained residue (13 g) is taken up with ethyl ether and stirred for 1 hour at -10°C. The reaction mixture is filtered, washing with a little cold ether. 5.8 g of the title compound is obtained.

Analysis:

m.p. = 118 ÷ 120 °C
I.R. (ν max) : 3300, 1600, 1550 $cm^{-1}$

| Elemental analysis : | | calculated | found |
|---|---|---|---|
| | C | 61.95 | 61.86 |
| | H | 10.69 | 10.71 |
| | N | 8.03 | 8.05 |

Example 22

3-hexyl-(7-azido-heptyl)-cyclopentyl-azide

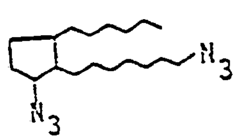

The compound of Example 3 (20 g) is handled as described in Example 7 using $CH_2Cl$ (120 ml), $N(C_2H_5)_3$ (33.8 ml),mesyl chloride (16.4 ml), water (190 ml), $NaN_3$ (14g), hexadeciltributylphosphonium bromide (4.4 g-) .After this reaction and chromatography,the title compound is obtained (18 g).
I.R. ν max): 2200 $cm^{-1}$.

Example 23

3-hexyl-2-(7-amino-heptyl)-cyclopentyl-amine. Derivative of formula (IA) wherein : n= 2; m = 5; p = 6; X = H; Y = $NH_2$;
Z = $CH_2NH_2$ (IBI-P-01068).

The compound of Example 22 (18 g) is handled as described in Example 11, using MeOH (300 ml), ethyl ether(50 ml) as co-solvent 5% pd/C (7.2 g).

At the end of the reaction and after silica gel chromatography and elution with 8 : 2 methylene chloride and methanol, 4.6 g of the title compound is obtained.

Analysis:

Titre as base = 90%
I.R. (ν max): 3300, 1670 $cm^{-1}$

| Elemental analysis : | | calculated | found |
|---|---|---|---|
| | C | 76.6 | 76.5 |
| | H | 13.47 | 13.41 |
| | N | 9.93 | 9.96 |

Example 24

3-hexyl-2-(6-carbomethoxy-hexyl)-cyclohexanone

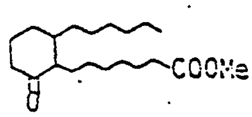

Into a suspension of Mg (4.5 g) in anhydrous THF (180 ml) is added dropwise bromohexane (30.5 g), refluxing the solvent until it is completely dissolved. The reaction mixture is cooled to 0°C, added with CuI (3.5 g) and stirred for 1 hour at 0°C until the formation of alkyl-copper is ended. The mixture is then cooled to -30°C and added dropwise, over 10 minutes, with a solution of 2-(carbomethoxyhexyl)-2-cyclohexene-1-one (21 g) dissolved in THF (30 ml). After 15 minutes at -30 ÷ -25°C, a saturated solution of $NH_4Cl$ in water, with PH adjusted to 8 with $NH_4OH$ (300 ml) is added rapidly. The reaction mixture is stirred from 30 minutes to 1 hour, at room temperature, and the phases are separated. The organic phase is washed to neutrality with a saturated sodium chloride solution and anhydrated The solvent is then evaporated and chromatographed on silica gel, eluting with 8 : 2 n-hexane/ethyl acetate, thus otaining the title compound (18 g).
I.R. (ν max): 1740, 1710 $cm^{-1}$.

Example 25

3-hexyl-2-(6-carboxy-hexyl)-cyclohexanone

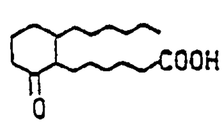

The compound of Example 24 (18 g) is dissolved in methanol (20 ml) and treated with an NaOH (4g) solution in water (80 ml). The reaction mixture is refluxed, under stirring, for 2 hours, it is cooled and acidified to pH 2, it is extracted with methylene chloride (80 ml) and washed with water to neutrality. The solvent is then anhydrated and evaporated. 15.5 g of the title compound is obtained.
I.R. : 3300, 1740, 1710 $cm^{-1}$

Example 26

3-hexyl-2-(6-carboxy-hexyl)-cyclohexyl-amine. Derivative of formula (IA) wherein n = 3; m = 5; p = 6; X = H; Y= $NH_2$;
Z = COOH (IBI-P-05006).

The compound of Example 25 is treated in an autocla e at 40 ÷ 50°C with methanol (200 ml), ammonia, (35 g), $PtO_2$ (0.150 g) and kept under hydrogen pressure ( 5.7 atm) for 3 days. Thereafter, 9.9 g of the title compound is obtained.

Analysis:

I.R.(v max): 3300,1550 $cm^{-1}$; m.p. = 161 ÷ 163 °C

| Elemental analysis : | | calculated | found |
|---|---|---|---|
| | C | 73.31 | 73.15 |
| | H | 12.54 | 12.29 |
| | N | 4.5 | 4.62 |

Titre as base = 97%.

Example 27

3-hexyl-2-(7-amino-heptyl)-cyclohexyl-amine. Derivative of formula (IA) wherein n = 3; m = 5; p = 6; X = H; Y = $NH_2$;
Z = $CH_2NH_2$ (IBI-P-05012).

This compound is prepared starting from 3-hexyl-2-(6-carboxy-hexyl)-cyclohexanone of Example 25 with a process analogous to that used for preparing the compound IBI-P-01068 (Examples 22 and 23).

Analysis:

I.R. (v max) : 3280, 1460 $cm^{-1}$
Titre as base = 91%.

Example 28

3-hexyl-2-(5-carbomethoxy-pentyl)-cyclohexanone

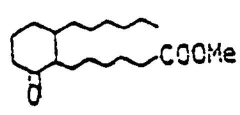

37 g of 2-(5-carbomethoxy-pentyl)-2-cyclohexene-1-one is handled as described in Example 24 using: bromohexane (54.5 g) Mg (7.92 g) THF (540 ml), CuI (12.3 g), a saturated $NH_4Cl$ solution at pH 8.4 (5S0 ml).At the end of the reaction and after chromatography on silica gel, 38.4 g of the title compound is obtained.

Analysis:

I.R. (v max) : 1740,1710 $cm^{-1}$
$^1$H-NMR: 3.7 (s, 3H); 2.3 (t, 2H); 1.4 ÷ 0.9 (broad,29H).

Example 29

3-hexyl-2-(5-carboxy-pentyl)-cyclohexanone

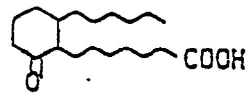

The compound of example 28 (20 g) is handled as described in Example 25, using methanol (25 ml), NaOH (5.1 g), water (52 ml). Thereafter 18.1 g of the title compound.
I.R.(ν max) : 3400 ÷ 3700, 1700 $cm^{-1}$.

Example 30

3-hexyl-2-(5-carboxy-pentyl)-cyclohexyl-amine. Derivative of formula (IA) wherein : n = 3; m = 5; p = 5; X = H; Y =$NH_2$;
Z = COOH (IBI-P-05008).

The compound of Example 29 (18.1 g) is treated in an autoclave,at room temperature, together with methanol (200 ml), ammonia (62 g),$PtO_2$ (0.18 g) and left under hydrogen pressure for 3 days . At the end of which and after crystallization from ethyl ether (100 ml) 12 g of the title compound is obtained.

Analysis:

I.R. (ν max) : 3400 ÷ 2800, 1550 $cm^{-1}$
m.p. : 155 ÷ 158 °C, with decomposition
Titre as base = 98%

Elemental analysis:

| | calculated | found |
|---|---|---|
| C | 72.67 | 72.42 |
| H | 11.86 | 11.68 |
| N | 4.70 | 4.59 |

Example 31

3-hexyl-2-(6-cyano-hexyl)-cyclohexyl-amine.Derivative of formula (IA) wherein : n = 3, m = 5; p = 6; X = H; Y = $NH_2$;
Z = CN (IBI-P-05009).

This compound is prepared starting from 3-hexyl-2-(6-carboxy-hexyl)-cyclohexane of Example 25, by an analogous process to that used for the preparation of the compound IBI-P-01037 (Examples 3,4,5,6,7,8).

Analysis:

I.R.(ν max) : 2220 $cm^{-1}$
$^1$H-NMR: 2.2 (t, 2H); 1.4 (s, 2H); 1.2 ÷ 0.9 (broad,31H)
Titre as base = 92%

Elemental analysis

| | calculated | found |
|---|---|---|
| C | 78.41 | 78.32 |
| H | 12.47 | 12.45 |
| N | 9.62 | 9.63 |

Example 32

5-spiro-[2-(7-carboxy-hexyl)-3-hexyl-cyclohexyl]-imidazoline-2,4-dione. Derivative of formula (IA) wherein : n = 3; m = 5; p = 6; X and Y, taken together, form a hydantoin nucleus;

Z = COOH (IBI-P-05010).

The compound of Example 25 (18 g) is dissolved in an NaOH solution (2.32 g) in water (90 ml) and handled as described in Example 2, using KCN (3.8 g), $(NH_4)_2CO_3$ (17 g), water (90 ml). Whereupon the title compound is obtained as a white crystalline solid (12.5 g).

Analysis:

I.R.(ν max) : 3250, 1720,1680 $cm^{-1}$
$^1$H-NMR: 8.3 (s, 1H); 2.36 (t, 2H),1.8 ÷ 0.9 (broad 33H)
Titre (acid) = 97%

Elemental analysis :

| | calculated | found |
|---|---|---|
| C | 66.28 | 65.75 |
| H | 9.53 | 9.73 |
| N | 7.35 | 7.26 |

Example 33

3-hexyl-2-(6-carbomethoxy-hexyl)-carbonyl-cyclohexane

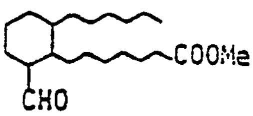

A solution of potassium ter.butylate (14.5 g) in DMSO (1.5 l) is added, under argon, with $Ph_3PClCH_2OMe$ (44.4 g) and, after 20 minutes, with the compound (28g) of Example 24,stirring for 16 hours.The reaction mixture is then poured into cold water (500 ml) and acidified with 6N HCl (30 ml), extracted with ethyl ether (500 ml) and washed up to neutrality with a 1:1 water/saturated NaCl solution. The solvent is anhydrated and evaporated to dryness, under reduced pressure, chromatographed on silica gel and eluted with 8 : 2 hexane/ethyl acetate, thus obtaining 23 g of a yellow oil which is taken up with acetone (500 ml) and 3N HCl (120 ml) and stirred for 1 hour. It is then diluted with water (300 ml) and extracted with ethyl ether (300 ml). The etheral phase is washed with a 1:1 water/saturated NaCl solution up to neutrality and anhydrated. The reaction mixture is evaporated under reduced pressure yielding 21 g of the title compound.
I.R. (ν max.):2720 , 1740 $cm^{-1}$

Example 34

3-hexyl-2-(6-carbomethoxy-hexyl)-hydroxymethyl-cyclohexane

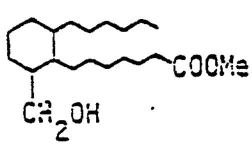

The compound of Example 33 (21 g) is dissolved in MeOH (200 ml) and treated, portionwise, with $NaBH_4$ (1.12 g) at 0° ÷+5°C. After 1 hour at 0° ÷ +5°C, the reaction mixture is slowly acidified with 3N HCl and the phases are extracted with ether (300 ml). The ethereal phase is washed with water up to neutrality an anhydrated. The solvent is evaporated under reduced pressure and the residue is chromatographed on silica gel, eluting with 8 : 2 hexare/ethyl acetate, thus obtaining 8 g of the title compound.
I.R. (ν max.): 3200 $cm^{-1}$, 1740 $cm^{-1}$.

Example 35

3-hexyl-2-(6-carbomethoxy-hexyl)-azidomethyl-cyclohexane

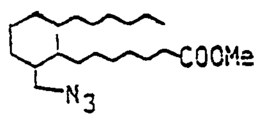

The compound of Example 34 (8 g) is treated as described in Example 7, using $CH_2Cl_2$ (100 ml),$N(C_2H_5)_3$ (4.95 ml), mesyl chloride (2.4 ml), $NAN_3$ (38 g), water (50 ml),hexadecyl-tributylphosphonium bromide(1.2 g),whereupon 8.6 g of the title product is obtained.
I.R. (ν max) : 2120, 1740 $cm^{-1}$

| Elemental analysis : | | calculated | found |
|---|---|---|---|
| | C | 69.00 | 88.91 |
| | H | 10.75 | 10.53 |
| | N | 11.40 | 11.24 |

Example 36

3-hexyl-2(6-carboxy-hexyl)-aminomethyl-cyclohexane. Derivative of formula (IA) wherein : n = 3; m = 5; p = 6; X = H; Y = $CH_2NH_2$; Z = COOH (IBI-P-05011).

The compound of Example 35 (8.3 g) is handled as described in Example 8, using methanol (160 ml), 5% Pd/C (1.1 g), whereafter a clear oil (7.4 g) is obtained which is taken up with methanol (25 g) and added to a solution of NaOH (1.49 g) in water (50 ml). The reaction mixture is heated at 65°C for 2 hours, under stirring, it is then cooled to room temperature and the pH is adjusted to 5.5 with 6N HCl. The reaction mixture is then stirred for 1 hour at +5°C, the solid thus obtained is filtered and washed first with a small amount of cold methanol and then with ether.It is filtered and dried under reduced pressure yielding 3.5 g of the title compound.

| Elemental analysis: | | calculated | found |
|---|---|---|---|
| | C | 73.79 | 73.78 |
| | H | 12.07 | 12.14 |
| | N | 4.3 | 4.39 |

Titre as base = 94%

Example 37

2-pentyl-3-(7-carboxy-heptyl)-cyclopentanol

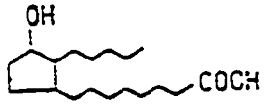

A solution of 2-(n-pentyl)-3-(carbomethoxy heptyl) cyclopentane-1-one (150 g) (prapared in like manner as described in IL FARMACO 27, 610 (1972)), in methanol (800 ml), is treated, portionwise, with $NaBH_4$ (24.1 g) at 0 ÷ +5°C. After 1.5 hours at 0 ÷ +5°C, the reaction mixture is poured into water (900 ml), acidified with 6N HCl and extracted with ethyl ether (1 l). The organic phase is washed with water to neutrality and anhydrated. The solvent is evaporated and 148 g of a clear oil is obtained.
I.R.(νmax) : 3200, 1735 $cm^{-1}$

Example 38

2-pentyl-3-(7-carboxy-heptyl)-cyclopentyl amine.Derivative of formula (IB) wherein : n = 2; m = 4; p = 7; X. = H; Y = $NH_2$; Z = COOH (IBI-P-10002).

The compound of Example 37 (148 g) is handled as described in Example 7, using $CH_2Cl_2$ (1.5 lt), $(C_2H_5)_3$ (109 ml), mesylchloride (54.8 ml) $NaN_3$ (64.5 g), water (1.3 l ), hexadecyl-tributylphosphonium bromide (23.5 g), whereupon 143 g of chromatographed compound (I.R.=2100 $cm^{-1}$) is obtained which is treated as described in Example 8, using methanol (1.5 lt), 5% Pd/C (24 g). 100 g of a clear oil is obtained which is treated as described in Example 19, using methanol (700 ml), $K_2CO_3$ (93 g), water (310 ml). Thus 48 g of the title compound is obtained in the form of a white crystalline solid.

| Elemental analysis : | | calculated | found |
|---|---|---|---|
| | C | 72.67 | 72.58 |
| | H | 11.86 | 11.83 |
| | N | 4.71 | 4.82 |

Example 39

N-[2-pentyl-3-(7-carboxy-heptyl)-cyclopentyl]-methyl-amine. Derivative of formula (IB) wherein : n = 2; m = 4; p = 7; X = H; Y = $NHCH_3$; Z = COOH (IBI-P-10004).

A solution of 2-(pentyl-3-(caboxy-heptyl)-cyclopentane-1-one (50 g) in methanol (100 ml) is added with a solution of NaOH (6.9 g) in methanol (100 ml). Said solution is treated in a 500 ml steel autoclave with $CH_3NH_2$ (53 ml) and $PtO_2$ (1 g) at -40°c. It is left under hydrogen(4 atm.)and heated at 50°C for 24 hours. The catalyst is then filtered off and desiccated,yielding 50.8 g of a semi-solid white compound. This residue is taken up with $CH_3CN$ (700 ml) for 3 hours at 0 ÷ +5°C, filtered off, yielding 50 g of the title compound in the form of a white crystalline solid.

Analysis:

m.p. : 200 ÷ 224 °C.
Titre as base = 98%
I.R. (ν max): 3300, 1500 $cm^{-1}$
$^1$H-NMR: 8.1 (s, 1H); 2.5 (s, 1H); 1.8 ÷ 0.9 (broad, 32 H).

Example 40

2-pentyl-3-(8-hydroxy-octyl)-cyclopentyl amine. Derivative of formula (IB) wherein : n = 2; m = 4; p = 7; X = H; Y = $NH_2$; Z = $CH_2OH$ (IBI-P-10007).

The methyl ester of the compound of Example 38 is handled as described in Example 1, using ethyl ether (300 ml), $LiAlH_4$ (2.85 g), whereupon 16.g of the title compound is obtained.

Analysis :

Titre as base = 96%
I.R. (ν max) : 3000, 1650 , 1460 $cm^{-1}$
$^1$H-NMR : 3.6 (t, 2H); 2.4 (s, 3H); 1.4 - 0.9(broad,32H).

Example 41

2-(6-carboxy-hexyl)-3-heptyl-cyclopentyl amine. Derivative of formula (IA) wherein: n = 2. m = 6; p = 6; X = H; Y = $NH_2$; Z = COOH (IBI-P-12004).

This derivative is prepared starting from 2-(carbomethoxy-hexyl)-cyclopentyl-2-ene-1-one by an analogous method to that used for preparing the compound IBI-P-10002 (Examples 37 and 38).

Analysis:

I.R. (ν max): 3300, 1560 $cm^{-1}$
Titre as base = 94%

| Elemental analysis: | | calculated | found |
|---|---|---|---|
| | C | 68.43 | 67.8 |
| | H | 10.88 | 10.51 |
| | N | 4.20 | 4.09 |

Example 42

2-(6,6-diethoxy-hexyl)-3-hexyl-cyclopentyl amine.Derivative of formula (IA) wherein n = 2; m = 5; p = 5; X = H; Y = $NH_2$; Z = $CH(OEt)_2$ (IBI-P-12007).

This compound is prepared starting from 2-(5-carbomethoxy-pentyl)-cyclopent-2-ene-1-one by an analogous method to that used for preparing the compound IBI-P-01053 (Examples 9,10 and 11).

Analysis :

I.R.(ν max) : 3340 $cm^{-1}$
$^1$H-NMR : 4.6 (t, 2H); 3.6 (m, 5H); 2 (s,2H); 1.9 ÷ 0.9 (broad, 30H).
Titre as base = 96%

| Elemental analysis : | | calculated | found |
|---|---|---|---|
| | C | 73.84 | 73.69 |
| | H | 12.69 | 12.58 |
| | N | 4.09 | 4.20 |

Example 43

2-(6,6-diethoxy-hexyl)-3-heptyl-cyclopentyl amine. Derivative of formula (IA) wherein: n = 2; m = 6; p = 5; X = H; Y = $NH_2$; Z =-$CH(OC_2H_5)_2$ (IBI-P-12008).

This derivative is prepared starting from 2-(5-carbomethoxy-pentyl)-cyclopent-2-ene-1-one by an analogous method to that used for preparing the compound IBI-P-01053 (Examples 9,10 and 11).

Analysis:

I.R. (ν max) : 3350 $cm^{-1}$
$^1$H-NMR : 4.4 (t,1H); 3.5 (m, 5H); 1.4÷0.9 (broad, 37H)
Titre as base = 95%

Example 44

5-spiro [2-(5-carboxy-pentyl)-3-heptyl-cyclopentyl]-imidazoline-2,4-dione.Derivative of formula (IA) wherein: n = 2; m = 6; p = 5; X and Y constitute the hydantoin nucleus; Z = COOH (IBI-P-12009).

This compound is prepared starting from 2-(5-carbomethoxy-pentyl)-cyclopent-2-ene-1-one by an analogous process as that used for preparing the compound IBI-P-01028 (Ex. 2).

Analysis :

I.R.(ν max) : 3700, 3100, 1770, 1740, 1720, 1460 $cm^{-1}$
$^1$ H-NMR : 9.5 (s, 1H); 7.3 (s, 1H); 2.3 (m, 4H); 1.4÷0.95 (broad, 27H).

Example 45

1-cyano-2-(6-carbomethoxy-hexyl)-3-hexyl-cyclopentyl amine

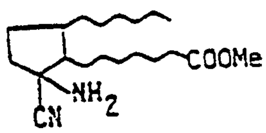

A solution of NaOH (4.55 g) in water (100 ml) is added, portionwise, with 9-oxo-19,20-bis-nor-prostanoic acid and left under stirring until it is completely dissolved. 30% $NH_4OH$ (150 ml), $NH_4Cl$ (10.1 g) and KCN (12.28 g) are then added at room temperature. The reaction mixture is stirred for 3 days at room temperature. It is then cooled to 0°C and cautiously acidified with concentrated hydrochloric acid so as to adjust the pH value at 4, and extracted with ethyl ether (500 ml). The ethereal extract is washed with water and anhydrated. Upon evaporation of the solvent under reduced pressure, a brown oil (29 g) is obtained which is taken up with methanol (300 ml), p-toluene-sulfonic acid (18.5 g) and the solution is refluxed for 2 hours. Whereupon the solvent is evaporated to small volume, poured into water (300 ml) and 10% NaOH (10 ml) and extracted with ethyl ether (300 ml). The etheral extract is washed with water to neutrality and anhydrated.

After evaporation and silica gel chromatography, eluting with 7 : 3 hexane/ethyl acetate, 12 g of the title compound is obtained.

Analysis :

I.R. (ν max) : 3320, 3350, 2220, 1740, 1620 $cm^{-1}$
Titre as base = 98%

Example 46

1-aminomethyl-2-(7-hydroxy-heptyl)-3-hexyl-cyclopentyl amine. Derivative of formula (IA) wherein : n = 2; m = 5; p = 6; X = $NH_2$; Y =$CH_2NH_2$; Z = $CH_2OH$ (IBI-P-01070).

To a suspension of $LiAlH_4$(3.42 g) in THF (90 ml), heated to reflux, is added dropwise a solution of compound of Example 45 (10 g) dissolved in THF (20 ml). The solution is refluxed for $2\frac{1}{2}$ hours and cooled to 0°C. Water (20 ml) and ethyl ether (100 ml) are then cautiously added dropwise.The phases are separated and the aqueous phase is extracted with ethyl ether (100 ml). The etheral phases are washed with water to neutrality and anhydrated. After evaporation and silica gel chromatography, eluting with 7 : 3 methylene chloride/methanol, the title product is obtained (3.5 g).

Analysis :

I.R.(ν max) : 3300, 1650, 1480 $cm^{-1}$
$^1$H-NMR : 3.6 (t, 2H); 2.5 (s, 2H); 2.1 (s, 5H); 1.7-0.9 (broad, 31H)
Titre as base = 98%.

## Claims

1. A compound of formula

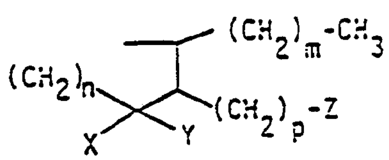
- (IA)

including the possible optical and/or geometric isomers wherein: n = 2, 3; m = 4, 5, 6, 7; p = 5, 6, 7; Y, when X=H, $-NH_2$, represents $-NH-NH_2$, $-CH_2NH_2$, -NRR′, wherein R and R′ may be the same or different, each representing: H, a linear or branched alkyl, alkenyl, alkynyl, phenyl, substituted aryl, or, taken together with the

substituent X, it may constitute the hydantoin nucleus and

Z = -CN, $-CH_2NH_2$, $-CH_2OH$, -CHO, 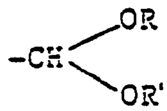 , $-COCH_3$,

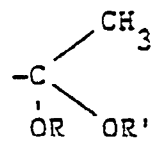 , COW,

wherein R and R′ have the meanings given above and W may be -OR, -NRR′, except for those compounds corresponding to the formula (IA) wherein: n = 2; m = 5, p = 6; X = H; Y= NRR′; and

Z = 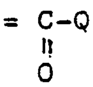

wherein Q = -OH, ($C_1$-$C_5$)-alkoxy, phenoxy, butyloxy, or -NHR″, wherein R″ is ($C_1$-$C_5$)alkyl or H.

2. A compound of formula

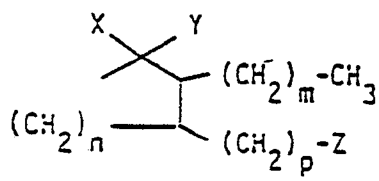 (IB)

including the possible optical and/or geometric isomers wherein: n = 2,3; m = 4,5,6,7; p = 5,6,7; Y, when X = H,-$NH_2$, represents -NH-$NH_2$, -$CH_2NH_2$, -NRR′, wherein R and R′ may be the same or different, each representing :
H, a linear or branched alkyl, alkenyl, alkynyl, phenyl, substituted aryl or, taken together with the substitutent X, it may constitute the hydantoin nucleus, and

Z = -CN, $-CH_2NH_2$, $-CH_2OH$, -CHO, 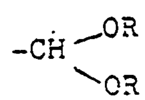 , $-COCH_3$, 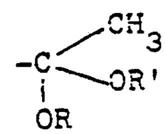 ,
COW,

wherein R and R′ have the meanings given above and W may be -OR, -NRR′.

3. A compound according to claim 1, wherein n = 2, m = 5, p = 6, Y =-$NH_2$, X = H and Z =-$CH_2OH$.

4. A compound according to claim 1, wherein: n = 2, m= 5, p = 6, Y and X, taken together, constitute the h dantoin nucleus and Z = -COOH.

5. A compound according to claim 1 wherein: n = 2, m = 5, p = 6, Y = -$NH_2$, X = H and Z = -CN.

6. A compound according to claim 1, wherein: n = 2, m = 5, p = 6, Y = -$NH_2$, X = H and Z=-$CH(OC_2H_5)_2$

7. A compound according to claim 1, wherein: n = 2, m = 5, p = 6, Y =-$NH_2$, X = H and Z=-$C(OC_2H_5)_2CH_3$.

8. A compound according to claim 1, wherein: n = 2, m = 5, p = 6, Y = -$CH_2NH_2$, X = H and Z = -COOH.

9. A compound according to claim 1, wherein: n = 2, m = 5, p = 6, Y = -$NHNH_2$; X = H and Z = -COOH.

10. A compound according to claim 1, wherein: n = 2, m = 5, p = 6, Y = -$NH_2$,X = H and Z = -$CH_2NH_2$.

11. A compound according to claim 1, wherein: n = 3, m = 5, p = 6, Y = -$NH_2$, X = H and Z = -COOH.

12. A compound according to claim 1, wherein: n = 3, m = 5, p = 5, Y = -$NH_2$, X = H and Z = -COOH.

13. A compound according to claim 1, wherein: n = 3, m = 5, p = 6, Y = -$NH_2$, X = H and Z = -CN.

14. A compound according to claim 1, wherein: n = 3, m = 5, p = 6, Y and X, taken together, constitute the hydantoin nucleus and Z = -COOH.

15. A compound according to claim 1, wherein: n = 3, m = 5, p = 6, Y = $-CH_2NH_2$, X = H and Z = -COOH.

16. A compound accordi,g to claim 1, wherein: n = 3, m = 5, p = 6, Y = $-NH_2$, X = H and Z = $-CH_2NH_2$.

17. A compound according to claim 1, wherein: n = 2, m = 6, p = 6, X = H, Y =$-NH_2$ and Z = COOH.

18. A compound according to claim 1, wherein: n = 2, m = 5, p = 5, Y = $-NH_2$, X = H and Z = $-CH(OC_2H_5)_2$.

19. A compound according to claim 1, wherein: n = 2, m = 6, p = 5, Y = $-NH_2$, X = H and Z = $-CH(OC_2H_5)_2$.

20. A compound according to claim 1, wherein: n = 2, m = 6, p = 5, Y and X, taken together, constitute the hydantoin nucleus and Z = -COOH.

21. A compound according to claim 2, wherein: n = 2, m = 4, p = 7, Y = $-NH_2$, X = H and Z = -COOH.

22. A compound according to claim 2, wherein: n = 2, m = 4, p = 7, Y = $-NHCH_3$, X = H and Z = -COOH.

23. A compound according to claim 2, wherein: n = 2, m = 4, p = 7, Y = $-NH_2$, X = H and Z = $CH_2OH$.

24. A compound according to claim 1, wherein: n = 2, m = 5, p = 6, Y = $-CH_2NH_2$, X = $NH_2$ and Z =$-CH_2OH$.

25. A process for preparing a compound of formula (IA) according to claim 1, wherein X is H and Y is the -NRR′ group,wherein R and R′ can be the same or different, each representing H, a linear or branched alkyl, alkenyl, alkynyl, phenyl or substituted aryl, characterized by one fact that a cyclic ketone of formula (IIIA), wherein the letters n, m, p, Z have the meanings indicated in formula (IA), is made to react with ammonia or with an -NHRR′ amine, in a polar organic solvent, under hydrogen atmosphere and in the presence of a metal catalyst, at a temperature ranging from 20°C to the reflux temperature of the reaction mixture, over a period of time from 1 to 100 hours.

26. A process for preparing a compound of formula (IB) according to claim 2, wherein X is H and Y is the -NRR′ group, wherein R and R′ can be the same or different, each representing H, a linear or branched alkyl,alkenyl, alkynyl, phenyl or substituted aryl, characterized by the fact that a cyclic ketone of the formula (IIIB), wherein the letters n, m, p and Z have the meanings indicated in the formula (IB), is made to react with ammonia or an -NHRR′ amine, in an organic polar solvent, under hydrogen atmosphere and in the presence of a metal catalyst, at a temperature ranging from 20°C to the reflux temperature of the reaction mixture, over a period of time of from 1 to 100 hours;

27. A process for preparing a compound of formula (IA) according to claim 1, wherein X and Y,taken together, constitute the hydantoin nucleus, characterized by the fact that a ketone of formula (IIIA), wherein the letters n, m, p and Z have the meanings indicated in formula (IA), is made to react with an alkali or alkaline earth metal cyanide, under controlled pH, in a polar solvent and at a temperature of from 20°C to the reflux temperature of the reaction mixture, over a period of time of from 1 to 20 hours.

28. A process for preparing a compound of formula (IB) according to claim 2, wherein X and Y, taken together, constitute the hydantoin nucleus, characterized by the fact that a ketone of formula (IIIB),wherein the letters n, m, p and Z have the meanings indicated in formula (IB), is made to react with an alkali or alkaline earth metal cyanide, under controlled pH, in a polar solvent and at a temperature of from 20°C to the reflux temperature of the reaction mixture, for a period of time of from 1 to 20 hours.

29. A pharmaceutical composition having anti-ulcer activity characterized by the fact that it contains, as the active ingredient, a compound of formula (IA) according to claim 1, or a pharmaceutically acceptable salt thereof.

30. A pharmaceutical composition having anti-ulcer activity characterized by the fact that it contains, as the active ingredient, a compound of formula (IB) according to claim 2, or a pharmaceutically acceptable salt thereof.

## Patentansprüche

1. Verbindungen der Formel

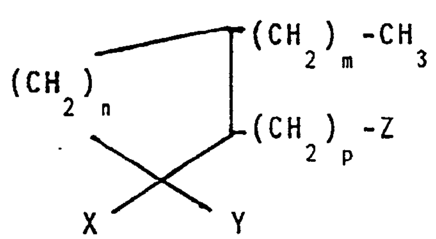

(IA) ,

einschließlich der möglichen optischen und/oder geometrischen Isomeren, in der n 2 oder 3 bedeutet ;

m 4, 5, 6 oder 7 bedeutet ;
p 5, 6 oder 7 bedeutet ;
Y, wenn X für H oder $-NH_2$ steht, $-NH-NH_2$, $-CH_2-NH_2$ oder NRR′ bedeutet, wobei $R^2$ und $R^1$ gleich oder verschieden sein können und jeweils H, geradkettiges oder verzweigtes Alkyl, Alkenyl, Alkinyl, Phenyl, substituiertes Aryl oder, zusammen mit dem Substituenten X, einen Hydantoin-Ring bedeuten; und

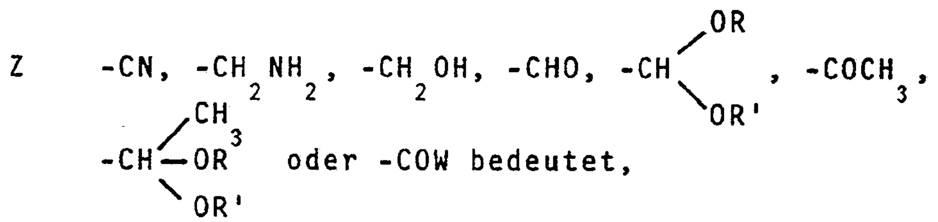

wobei R und R′ die oben angegebene Bedeutung haben und W -OR oder -NRR′ bezeichnet ;
ausgenommen die Verbindungen der Formel (IA), in denen n = 2, m = 5, p = 6 ist ; X für H und Y für -NRR′ steht; und

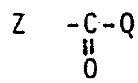

bedeutet, wobei Q für -OH, ($C_1$-$C_5$)-Alkoxy, Phenoxy, Butyloxy oder den Rest -NHR″steht, in dem R″ ($C_1$-$C_5$)-Alkyl oder H bezeichnet.

2. Verbindungen der Formel

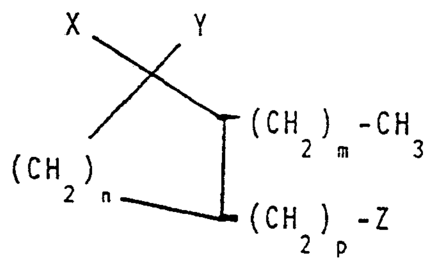

(IB) ,

einschließlich der möglichen optischen und/oder geometrischen Isomeren, in der
n 2 oder 3 bedeutet ;
m 4, 5, 6 oder 7 bedeutet ;
p 5, 6 oder 7 bedeutet ;
Y, wenn X für H oder $-NH_2$ steht, $-NH-NH_2$ , $-CH_2$ $-NH_2$ oder -NRR′ bedeutet, wobei R und R′ gleich oder verschieden sein können und jeweils H, geradkettiges oder verzweigtes Alkyl, Alkenyl, Alkinyl, phenyl, substituiertes Aryl oder, zusammen mit dem Substituenten X, einen Hydantoin-Ring bedeuten ; und

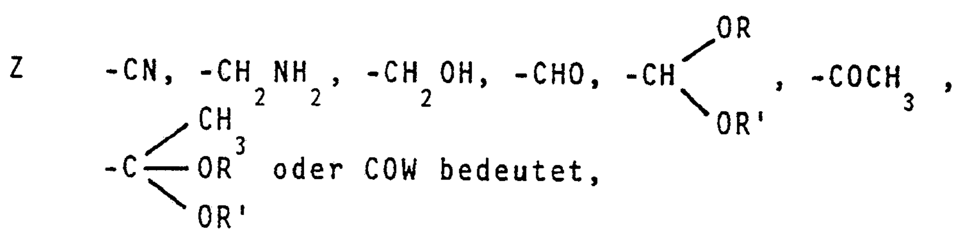

worin R und R′die oben angegebenen Bedeutungen haben und W -OR oder -NRR′ bezeichnet.

3. Verbindung nach Anspruch 1, in der n für 2, m für 5, p für 6, Y für $-NH_2$ , X für H und Z für $-CH_2$ OH steht.

4. Verbindung nach Anspruch 1, in der n für 2, m für 5, p für 6, Y und X zusammen für einen Hydantoin-Ring stehen und Z für -COOH steht.

5. Verbindung nach Anspruch 1, in der n für 2, m für 5, p für 6, Y für $-NH_2$, X für H und Z für -CN steht.

6. Verbindung nach Anspruch 1, in der n für 2, m für 5, p für 6, Y für $-NH_2$, X für H und Z für $-CH(OC_2H_5)_2$ steht.

7. Verbindung nach Anspruch 1, in der n für 2, m für 5, p für 6, Y für $-NH_2$, X für H and Z für $-C(OC_2H_5)_2CH_3$ steht.

8. Verbindung nach Anspruch 1, in der n für 2, m für 5, p für 6, Y für $-CH_2NH_2$, X für H und Z für -COOH steht.

9. Verbindung nach Anspruch 1, in der n für 2, m für 5, p für 6, Y für $-NH-NH_2$, X für H und Z für -COOH steht.

10. Verbindung nach Anspruch 1, in der n für 2, m für 5, p für 6, Y für $-NH_2$, X für H und Z für $-CH_2NH_2$ steht.

11. Verbindung nach Anspruch 1, in der n für 3, m für 5, p für 6, Y für $-NH_2$, X für H und Z für -COOH steht.

12. Verbindung nach Anspruch 1, in der n für 3, m für 5, p für 5, Y für $-NH_2$, X für H und Z für -COOH steht.

13. Verbindung nach Anspruch 1, in der n für 3, m für 5, p für 6, Y für $-NH_2$, X für H und Z für -CN steht.

14. Verbindung nach Anspruch 1, in der n für 3, m für 5, p für 6, Y und X zusammen für einen Hydantoin-Ring stehen und Z für -COOH steht.

15. Verbindung nach Anspruch 1, in der n für 3, m für 5, p für 6, y für $-CH_2NH_2$, X für H und Z für -COOH steht.

16. Verbindung nach Anspruch 1, in der n für 3, m für 5, p für 6, Y für $-NH_2$, X für H und Z für $-CH_2NH_2$ steht.

17. Verbindung nach Anspruch 1, in der n für 2, m für 6, p für 6, X für H, Y für $-NH_2$ und Z für -COOH steht.

18. Verbindung nach Anspruch 1, in der n für 2, m für 5, p für 5, Y für $-NH_2$, X für H und Z für $-CH(OC_2H_5)_2$ steht.

19. Verbindung nach Anspruch 1, in der n für 2, m für 6, p für 5, Y für $-NH_2$, X für H und Z für $-CH(OC_2H_5)_2$ steht.

20. Verbindung nach Anspruch 1, in der n für 2, m für 6, p für 5, Y und X zusammen für einen Hydantoin-Ring stehen und Z für -COOH steht.

21. Verbindung nach Anspruch 2, in der n für 2, m für 4, p für 7, Y für $-NH_2$, X für H und Z für -COOH steht.

22. Verbindung nach Anspruch 2, in der n für 2, m für 4, p für 7, Y für $-NHCH_3$, X für H und Z für -COOH steht.

23. Verbindung nach Anspruch 2, in der n für 2, m für 4, p für 7, Y für $-NH_2$, X für H und Z für $-CH_2OH$ steht.

24. Verbindung nach Anspruch 1, in der n für 2, m für 5, p für 6, Y für $-CH_2NH_2$, X für $NH_2$ und Z für $-CH_2OH$ steht.

25. Verfahren zur Herstellung von Verbindungen der Formel (IA) nach Anspruch 1, in der X für H steht und Y die Gruppe -NRR′ bezeichnet, in der R und R′ gleich oder verschieden sein können und jeweils H, geradkettiges oder verzweigtes Alkyl, Alkenyl, Alkinyl, Phenyl oder substituiertes Aryl bedeuten, **dadurch gekennzeichnet**, daß ein cyclisches Keton der Formel (IIIA), in der n, m, p und Z die für die Formel (IA) angegebene Bedeutung haben, mit Ammoniak oder mit einem Amin der Formel NHRR′ in einem polaren organischen Lösungsmittel unter Wasserstoffatmosphäre und in Gegenwart eines Metallkatalysators bei einer Temperatur zwischen 20 °C und Rückflußtemperatur des Reaktionsgemisches und bei einer Reaktionszeit von 1 bis 100 Stunden umsetzt.

26. Verfahren zur Herstellung von Verbindungen der Formel (IB) nach Anspruch 2, in der X für H steht und Y die Gruppe -NRR′ bezeichnet, in der R und R′ gleich oder verschieden sein können und jeweils H, geradkettiges oder verzweigtes Alkyl, Alkenyl, Alkinyl, Phenyl oder substituiertes Aryl bedeuten, **dadurch gekennzeichnet**, daß ein cyclisches Keton der Formel (IIIB), in der n, m, p, und Z die für die Formel (IB) angegebene Bedeutung haben, mit Ammoniak oder einem Amin der Formel NHRR′ in einem polaren organischen Lösungsmittel unter Wasserstoffatmosphäre und in Gegenwart eines Metallkatalysators bei einer Temperatur zwischen 20 °C und Rückflußtemperatur des Reaktionsgemisches und bei einer Reaktionszeit von 1 bis 100 Stunden umsetzt.

27. Verfahren zur Herstellung von Verbindungen der Formel (IA) nach Anspruch 1, in der X und Y zusammen einen Hydantoin-Ring bedeuten, **dadurch gekennzeichnet**, daß ein Keton der Formel (IIIA), in der n, m, p, und Z die für die Formel (IA) angegebene Bedeutung haben, mit einem Alkali- oder Erdalkalicyanid bei kontrolliertem pH-Wert in einem polaren Lösungsmittel bei einer Temperatur zwischen 20 °C und Rückflußtemperatur des Reaktionsgemisches und bei einer Reaktionszeit von 1 bis 20 Stunden umgesetzt wird.

28. Verfahren zur Herstellung von Verbindungen der Formel (IB) nach Anspruch 2, in der X und Y zusammen einen Hydantoinring bedeuten, **dadurch gekennzeichnet**, daß ein Keton der Formel (IIIB), in der n, m, p und Z die für die Formel (IB) angegebene Bedeutung haben, mit einem Alkali- oder Erdalkalicyanid bei kontrolliertem pH-Wert in einem polaren Lösungsmittel bei einer Temperatur zwischen 20 °C und der Rückflusstemperatur des Reaktionsgemisches und bei einer Reaktionszeit von 1 bis 20 Stunden umgesetzt wird.

29. Pharmazeutische Zubereitungen mit Wirksamkeit gegen Geschwüre, **dadurch gekennzeichnet**, daß sie als Wirkstoff eine Verbindung der Formel (IA) nach Anspruch 1 oder deren pharmazeutisch akzeptables Salz enthalten.

30. Pharmazeutische Zubereitungen mit Wirksamkeit gegen Geschwüre, **dadurch gekennzeichnet**, daß sie als Wirkstoff eine Verbindung der Formel (IB) nach Anspruch 2 oder deren pharmazeutisch akzeptables Salz enthalten.

## Revendications

1. Composé de formule :

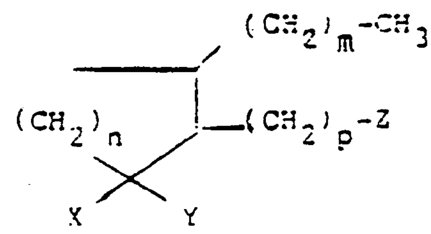

(IA)

y compris les isomères optiques et/ou géométriques possibles où n = 2, 3; m = 4, 5, 6, 7; p = 5, 6, 7; Y, lorsque X = H, $-NH_2$, représente $-NH-NH_2$, $-CH_2NH_2$, -NRR′, où R et R′ peuvent être identiques ou différents et représentent chacun H, alcynyle, alcényle, alcoyle linéaire ou ramifié, phényle, aryle substitué ou, pris ensemble avec le substituant X, peuvent constituer le noyau hydantoïque, et Z = -CN, $-CH_2NH_2$, $-CH_2OH$, -CHO, -CH ( OR) OR,′ $-COCH_3$, -C (OR) (OR′) $CH_3$; -COW;
où R et R′ ont les significations attribuées ci-dessus et W peut être -OR, -NRR′, excepté pour les composés correspondants de formule (IA), où n = 2; m = 5; p = 6; X = H; Y = NRR′, et Z = -C(O)-Q, où Q = OH, $(C_1-C_5)$-alcoxy, phénoxy, butyloxy ou -NHR″, où R″ est $(C_1-C_5)$ -alcoyle ou H.

2. Composé de formule :

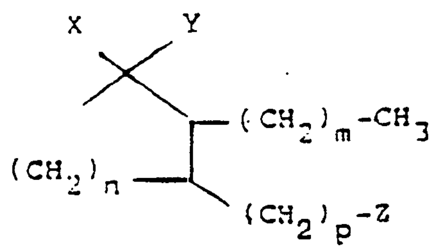

(IB)

y compris les isomères optiques et/ou géométriques possibles,
où n = 2, 3; m = 4, 5, 6, 7; p = 5, 6, 7; Y, lorsque X = H, $-NH_2$, représente $-NH-NH_2$, $-CH_2NH_2$, -NRR′, où R et R′ peuvent être identiques ou différents et représentent chacun : H, alcynyle, alcényle, alcoyle linéaire ou ramifié, phényle, aryle substitué ou, pris ensemble avec le substituant X, peuvent constituer le noyau hydantoïne et Z = -CN, $-CH_2NH_2$, $-CH_2OH$, -CHO, -CH(OR)OR′, $-COCH_3$, $-C(OR)(OR')CH_3$, -COW, où R et R′ ont les significations attribuées ci-dessus et W peut être -OR, -NRR′.

3. Composé suivant la revendication 1, dans lequel n = 2; m = 5; p = 6; Y = $-NH_2$; X = H et Z = $-CH_2OH$.

4. Composé suivant la revendication 1, dans lequel n = 2; m = 5; p = 6; Y et X, pris ensemble, constituent le noyau hydantoïne et Z = -COOH.

5. Composé suivant la revendication 1, dans lequel n = 2; m = 5; p = 6; Y = $-NH_2$; X = H et Z = -CN.

6. Composé suivant la revendication 1, dans lequel n = 2; m = 5; p = 6; Y = $-NH_2$; X = H et Z = $-CH(OC_2H_5)_2$.

7. Composé suivant la revendication 1, dans lequel n = 2; m = 5; p = 6; Y = $-NH_2$; X = H et Z = $-C(OC_2H_5)_2CH_3$.

8. Composé suivant la revendication 1, dans lequel n = 2; m = 5; p = 6; Y = $-CH_2NH_2$; X = H et Z = -COOH.

9. Composé suivant la revendication 1, dans lequel n = 2; m = 5; p = 6; Y = $-NHNH_2$; X = H et Z = -COOH.

10. Composé suivant la revendication 1, dans lequel n = 2; m = 5; p = 6; Y = $-NH_2$; X = H et Z = $-CH_2NH_2$.

11. Composé suivant la revendication 1, dans lequel n = 3; m = 5; p = 6; Y = $-NH_2$; X = H et Z = -COOH.

12. Composé suivant la revendication 1, dans lequel n = 3; m = 5; p = 6; Y = $-NH_2$; X = H et Z = -COOH.

13. Composé suivant la revendication 1, dans lequel n = 3; m = 5; p = 6; Y = $-NH_2$; X = H et Z = -CN.

14. Composé suivant la revendication 1, dans lequel n = 3; m = 5; p = 6; Y et X, pris ensemble, constituent le noyau hydantoïne et Z = -COOH.

15. Composé suivant la revendication 1, dans lequel n = 3; m = 5; p = 6; Y = $-CH_2NH_2$; X = H et Z = -COOH.

16. Composé suivant la revendication 1, dans lequel n = 3; m = 5; p = 6; Y = $-NH_2$; X = H et Z = $-CH_2NH_2$.

17. Composé suivant la revendication 1, dans lequel n = 2; m = 6; p = 6; X = H; Y = $-NH_2$ et Z = -COOH.

18. Composé suivant la revendication 1, dans lequel n = 2; m = 5; p = 5; Y = $-NH_2$; X = H et Z = $-CH(OC_2H_5)_2$.

19. Composé suivant la revendication 1, dans lequel n = 2; m = 6; p = 5; Y = $-NH_2$; X = H et Z = $-CH(OC_2H_5)_2$.

20. Composé suivant la revendication 1, dans lequel n = 2; m = 6; p = 5; Y et X, pris ensemble, constituent le noyau hydantoïne et Z = -COOH.

21. Composé suivant la revendication 2, dans lequel n = 2; m = 4; p = 7; Y = $-NH_2$; X = H et Z = -COOH.

22. Composé suivant la revendication 2, dans lequel n = 2; m = 4; p = 7; Y = $-NHCH_3$; X = H et Z = -COOH.

23. Composé suivant la revendication 2, dans lequel n = 2; m = 4; p = 7; Y = $-NH_2$; X = H et Z = $CH_2OH$.

24. Composé suivant la revendication 1, dans lequel n = 2; m = 5; p = 6; Y = $-CH_2NH_2$; X = $NH_2$ et Z = $-CH_2OH$.

25. Procédé de préparation d'un composé de formule (IA) suivant la revendication 1, où X est H et Y est le radical -NRR′, où R et R′ peuvent être identiques ou différents et représentent chacun H, alcynyle, alcényle, alcoyle linéaire ou ramifié, phényle ou aryle substitué, caractérisé en ce qu'une cétone cyclique de formule (IIIA), où les lettres n, m, p et Z ont les significations attribuées dans la formule (IA), est mise à réagir avec l'ammoniaque ou avec une amine -NHRR′ dans un solvant organique polaire, sous atmosphère d'hydrogène et en présence d'un métal catalytique, à une température s'échelonnant de 20°C à la température de reflux du mélange de réaction, pendant une durée de 1 à 100 heures.

26. Procédé de préparation d'un composé de formule (IB) suivant la revendication 2, où X est H et Y est le radical -NRR′, où R et R′ peuvent être identiques ou différents et représentent chacun H, alcynyle, alcényle, alcoyle linéaire ou ramifié, phényle ou aryle substitué, caractérisé en ce qu'une cétone cyclique de formule (IIIB), où les lettres n, m, p et Z ont les significations attribuées dans la formule (IB), est mise à réagir avec l'ammoniaque ou une amine -NHRR′ dans un solvant organique polaire, sous atmosphère d'hydrogène et en présence d'un métal catalytique, à une température s'échelonnant de 20°C à la température de reflux du mélange de réaction, pendant une durée de 1 à 100 heures.

27. Procédé de préparation d'un composé de formule (IA) suivant la revendication 1, où X et Y, pris ensemble, constituent le noyau hydantoïne, caractérisé en ce qu'une cétone de formule (IIIA), où les lettres n, m, p et Z ont les significations attribuées dans la formule (IA), est mise à réagir avec un cyanure de métal alcalin ou alcalino-terreux à pH imposé dans un solvant polaire et à une température s'échelonnant de 20°C à la température de reflux du mélange de réaction pendant une durée de 1 à 20 heures.

28. Procédé de préparation d'un composé de formule (IB) suivant la revendication 2, où X et Y, pris ensemble, constituent le noyau hydantoïne, caractérisé en ce qu'une cétone de formule (IIIB), où les lettres n, m, p et Z ont les significations attribuées dans la formule (IB), est mise à réagir avec un cyanure de métal alcalin ou alcalino-terreux à pH imposé dans un solvant polaire et à une température s'échelonnant de 20°C à la température de reflux du mélange de réaction pendant une durée de 1 à 20 heures.

29. Composition pharmaceutique ayant une activité antiulcéreuse, caractérisée en ce qu'elle contient comme constituant actif un composé de formule (IA) suivant la revendication 1 ou un sel pharmaceutiquement acceptable de celui-ci.

30. Composition pharmaceutique ayant une activité antiulcéreuse, caractérisée en ce qu'elle contient comme constituant actif un composé de formule (IB) suivant la revendication 2 ou un sel pharmaceutiquement acceptable de celui-ci.